# EUROPEAN PATENT SPECIFICATION

(11) **EP 4 120 966 B1**
(45) Date of publication and mention of the grant of the patent: **26.06.2024**
(21) Application number: 21711391.9
(22) Date of filing: 10.03.2021
(51) Int. Cl.: A61F 5/01, A63B 21/04, A61F 5/02, A61H 1/02

(54) **WEARABLE SUPPORT STRUCTURE FOR AT LEAST PARTLY RELIEVING A HUMAN BODY DURING LEANING OR BENDING OVER**
TRAGBARE STÜTZSTRUKTUR ZUR ZUMINDEST TEILWEISEN ENTLASTUNG EINES MENSCHLICHEN KÖRPERS BEIM NACHNEIGEN ODER UMBIEGEN
STRUCTURE SUPPORT PORTABLE POUR SOULAGER AU MOINS PARTIELLEMENT UN CORPS HUMAIN PENCHÉ VERS L'AVANT OU FLÉCHI

(30) Priority: 18.03.2020 NL 2025157
(43) Date of publication of application: 25.01.2023
(73) Proprietor: Laevo B.V., 2289 EX Rijswijk (NL)
(72) Inventor: WISSE, Boudewijn Martin, 2289 EX RIJSWIJK (NL); HÖLSCHER, Michael Martinus, 2289 EX RIJSWIJK (NL); WAGEMAKER, Sebastiaan Johannes, 2289 EX RIJSWIJK (NL)
(74) Representative: Algemeen Octrooi- en Merkenbureau B.V.
(86) International application number: PCT/NL2021/050160
(87) International publication number: WO 2021/187973

(56) References cited:
- WO-A1-2019/074350
- WO-A1-2019/139999
- US-A- 4 829 989
- US-A1- 2008 161 738
- US-A1- 2017 232 617
- US-A1- 2020 069 453

## Description

The present disclosure relates to a wearable support structure for at least partly relieving a human body during leaning or bending over, said wearable support structure comprising:
- a torso support arranged for bearing upon a chest and/or a back of said human body;
- two elongated resilient stays, connected to said torso support, arranged to extend substantially alongside said human body and arranged for being in a first state when being unloaded, and arranged to be biased back to said first state when being into a second state corresponding with a leaning or bending position of said human body;
- a lumbar support arranged for bearing upon a lumbar area of said human body;
- two thigh supports, each arranged for bearing upon a respective thigh area of said human body, preferably a front side of a respective thigh area of said human body;
- two pivoting arrangements, each connecting a respective elongated resilient stay of said two elongated resilient stays to a respective thigh support of said two thigh supports and allowing said respective elongated resilient stay and said respective thigh support to pivot about a main axis.

NL2014451 discloses a wearable support structure. One of the downsides of the wearable support structure disclosed by NL2014451 is that the known wearable support structure is relatively difficult to adapt to different levels of support of the human body. Using the known wearable support structures a human body is also biased back, by the two elongated resilient stays, to an upright position in situations wherein such biasing back is not desired, or at least desired to a different extent. Examples of such situations may occur when the wearer of the support structure is walking. During walking the biasing back to the upright position may be perceived as rather uncomfortable in that the chest support keeps pressing against the human body at a relative large biasing force. As such, a user wearing the known wearable support structure may feel hindered or restricted to walk while wearing the known wearable support structure.

Document WO2019/074350 discloses a wearable support structure according to the preamble of claim 1.

It is therefore an object of the present invention, which is defined by independent claim 1, to provide for a wearable support structure that is relative comfortable for the user during walking.

The object is achieved, in that the wearable support structure according to the present disclosure comprises a movement arrangement arranged for maintaining said two elongated resilient stays in said first state when said human body is in said upright position. By providing the movement arrangement it is avoided that the two elongated resilient stays are in said second state when said human body is in an upright position. The movement arrangement realises that the two elongated resilient stays are in said second position only when said human body is leaning or bending. This allows the user to move, for instance during walking, in the upright position without experiencing a biasing force, or at least experiencing only a relative small biasing force.

An additional advantage of providing the movement arrangement is that there is no need for a user of the wearable support structure according to the present disclosure to actively bring the wearable support structure in a condition wherein the user may move freely without experiencing the biasing force.

The movement arrangement accommodates for out-of-phase movements of the user of the wearable support structure in the sagittal plane of the human body.

Within the context of the present disclosure, the chest and the back of the human body referred to in relation to the torso support being arranged for bearing upon the chest and/or the back of said human body are to be understood as a part of the human body between the shoulders and the lumbar area of the human body. In other words, when a user is wearing the wearable support structure the torso support bears upon a part of the human body that is between the shoulders and the lumbar area of the user.

Within the context of the present disclosure, the lumbar area is to be understood as a part of the human body between the upper side of thighs and lower part of the back of the human body. The lumbar area may for instance refers to the hip, buttocks and an area of the back of the human body in proximity of the lower spine.

Preferably, said two thigh supports are arranged for being in a first state when being unloaded, and arranged to be biased back to said first state when being into a second state corresponding with a leaning or bending position of said human body. This is beneficial for providing a wearable support structure that is relative comfortable for the user during walking.

In this regard, it is advantageous if said movement arrangement is arranged for maintaining said two thigh supports in said first state when said human body is in said upright position. This is beneficial for providing a wearable support structure that is relative comfortable for the user during walking.

In an embodiment of the wearable support structure according to the present disclosure, said movement arrangement is arranged for maintaining a pivot angle about said main axis of said respective elongated resilient stay of said two elongated resilient stays substantially equal, preferably equal, to a pivot angle about said main axis of said respective thigh support of said two thigh supports when said human body is in said upright position. In other words, the movement arrangement is arrange for realising that the elongated resilient stay and the thigh support at a side of the human body pivot over the substantially the same angle about the main axis of the pivoting structure connecting the elongated resilient stay and the thigh support when the user of the wearable support structure is walking and thereby allowing the user to walk without experiencing a biasing force, or at least experiencing only a relative small biasing force.

Preferably, said movement arrangement comprises a first pivot structure, wherein said torso support is pivotally connected, via said first pivot structure to said two elongated resilient stays. Providing the first pivot structure is beneficial for allowing a movement between the torso support and the two elongated resilient stays. Such a movement between the torso support and the two elongated resilient states allows for the torso of the human body to move relative to the two elongated resilient stays while maintaining the two elongated resilient stays in the first state. This is beneficial for providing a wearable support structure that is relative comfortable for the user during walking.

In this regard, it is beneficial if said first pivot structure is arranged for pivoting said torso support relative to said two elongated resilient stays about a first pivot axis and a second pivot axis, wherein said first pivot axis is at right angles to said second pivot axis. This is beneficial for providing a wearable support structure that is relative comfortable for the user during walking.

Preferably, said first pivot axis, when said torso support is bearing upon said chest and/or said back of said human body, extends in a direction comprising a component in a vertical direction when said human body is in said upright position. In other words, the first pivot axis comprises a component in a direction that extends in a longitudinal direction of the human body. Within the context of the present disclosure, the longitudinal direction of the human body is to be understood as a direction perpendicular to the ground surface when the human body is in the upright position.

In this regard, it is beneficial if said first pivot structure is further arranged for pivoting said torso support relative to said two elongated resilient stays about a third pivot axis, wherein said third pivot axis is at right angles to said first pivot axis and said second pivot axis. Allowing said elongated resilient stays to pivot about the third pivot axis relative to the torso support is beneficial for reducing wringing forces that may be present in the wearable support structure during use of the wearable support structure. This is beneficial for providing a wearable support structure that is relative comfortable for the user during walking.

Preferably, said first pivot structure comprises an omnidirectional pivot coupling, preferably a ball-joint coupling, for allowing omnidirectional pivoting of said torso support relative to said two elongated resilient stays. This is beneficial for providing a wearable support structure that is relative comfortable for the user during walking.

In this regard, it is beneficial if said ball-joint coupling is arranged for pivoting of said torso support relative to said two elongated resilient stays about said first, second and/or third pivot axis.

In an embodiment of the wearable support structure according to the present disclosure, said omnidirectional pivot coupling comprises a flexible element for allowing said omnidirectional pivoting of said torso support relative to said two elongated resilient stays.

It is beneficial if said first pivot structure comprises a translation arrangement arranged for allowing said torso support to translate relative to said two elongated resilient stays. A translation arrangement is beneficial for avoiding, or at least significantly reducing, transfer of a shear force between the torso support and the human body. Such shear forces may be induced by tilt and rotation of the hip during gait of the human body. Allowing the torso support to translate relative to the two elongated resilient stays allows the torso support to bear upon the chest and/or back of the human body while avoiding, or at least significantly reducing, a movement of the torso support relative to the chest and/or back of the human body during gait of the user. This is beneficial for providing a wearable support structure that is relative comfortable for the user during walking.

In this regard, it is advantageous if said translation arrangement comprises a further omnidirectional pivot coupling, preferably a further ball-joint, for allowing said torso support to translate relative to said two elongated resilient stays, preferably for allowing said torso support to translate over a predetermined distance relative to said two elongated resilient stays. Providing a further ball-joint is beneficial for realising a wearable support structure wherein said torso support, when said two elongated resilient stays are in said second state, is biased back into a first position relative to said two elongated resilient stays. This is beneficial for providing a wearable support structure that is relative comfortable for the user during walking while realising a biasing back of the two elongated resilient stays that is relative comfortable for the user.

Preferably, said translation arrangement is arranged for translating said torso support in two perpendicular directions in a flat virtual plane relative to said two elongated resilient stays. This is beneficial for avoiding, or at least significantly reducing, transfer of a shear force between the torso support and the human body acting in the flat virtual plane. This is beneficial for providing a wearable support structure that is relative comfortable for the user during walking.

It is advantageous if said movement arrangement is arranged for allowing said two elongated resilient stays to pivot independent from each other relative to said torso support. This is beneficial for providing a wearable support structure that is relative comfortable for the user during walking.

Preferably, said movement arrangement is arranged for allowing said two elongated resilient stays to pivot independent from each other relative to said torso support about said second pivot axis.

Preferably, said movement arrangement further comprises a second pivot structure for allowing each of said elongated resilient stays to pivot about a fourth pivot axis relative to said pivoting arrangement connecting said elongated resilient stay, via said pivoting arrangement, to said thigh support, wherein said fourth pivot axis extends along said elongated resilient stay.

In this regard, it is beneficial if said fourth pivot axis is perpendicular to said main axis.

Preferably, the first pivot axis is substantially parallel, preferably parallel, to the fourth pivot axis. This is beneficial for allowing for a relative large stroke of the legs of the user in the upright position while maintaining the two elongated resilient stays in the first state. This is beneficial for providing a wearable support structure that is relative comfortable for the user during walking.

Preferably, said fourth pivot axis is provided outside said first pivot structure. This is beneficial for allowing for a relative large stroke of the legs of the user in the upright position while maintaining the two elongated resilient stays in the first state. This is beneficial for providing a wearable support structure that is relative comfortable for the user during walking.

In an embodiment of the wearable support structure according to the present disclosure, said main axes of said pivoting arrangements are concentric. This is beneficial for allowing for a relative large stroke of the legs of the user in the upright position while maintaining the two elongated resilient stays in the first state. This is beneficial for providing a wearable support structure that is relative comfortable for the user during walking.

It is beneficial if said wearable support structure comprises a third pivot structure, wherein said lumbar support is pivotally connected, via said third pivot structure, to said two pivoting arrangements. This is beneficial for providing a wearable support structure that is relative comfortable for the user during walking.

In this regard, it is beneficial if said third pivot structure is arranged for pivoting said lumbar support relative to said two pivoting arrangements about a fifth pivot axis. This is beneficial for providing a wearable support structure that is relative comfortable to wear during walking.

Preferably, said fifth pivot axis is concentric with said main axes. This is beneficial for providing a wearable support structure that is relative comfortable for the user during walking.

Preferably, said second pivot structure is arranged for pivoting said elongated resilient stays about a sixth pivot axis relative to said pivoting arrangements. Allowing said elongated resilient stays to pivot about a sixth pivot axis relative to said pivoting arrangements is beneficial for reducing wringing forces that may be present in the wearable support structure during use of the wearable support structure. This is beneficial for providing a wearable support structure that is relative comfortable for the user during walking.

Preferably, said sixth pivot axis is substantially at right angles, preferably at right angles, to the main axis and the fourth axis.

In an embodiment of the wearable support structure according to the present disclosure, said two pivoting arrangements are arranged for allowing said respective elongated resilient stay, connected to a respective pivoting arrangement of said two pivoting arrangements, to pivot about said main axis relative to said respective thigh support, connected to said respective pivoting arrangement of said two pivoting arrangements, wherein each of said two pivoting arrangements is arranged to provide a biasing force biasing said respective elongated resilient stay into a predetermined angle relative to said respective thigh support, wherein said movement arrangement is arranged for maintaining said respective elongated resilient stay into said predetermined angle relative to said respective thigh support and maintaining said two elongated resilient stays in said first state when said human body is in said upright position.

Preferably, said predetermined angle is in the range of 150 to 180 degrees.

In an embodiment of the wearable support structure according to the present disclosure, said wearable support structure comprises a fourth pivot structure for pivoting said thigh supports relative to said pivoting arrangements about a seventh pivot axis for allowing a user of said wearable support structure to move said thigh areas away from each other in a direction along said main axis.

The present invention will now be explained by means of a description of preferred embodiments of a wearable support structure according to the present disclosure, in which reference is made to the following schematic figures, in which:
Fig. 1: a side view of human bodies wearing a wearable support structure according to the present disclosure are shown;
Fig. 2: a top view of the human bodies from Fig. 1 are shown;
Fig. 3: a back view of the human bodies from Fig. 1 are shown;
Fig. 4: an isometric view of the human bodies from Fig. 1 are shown;

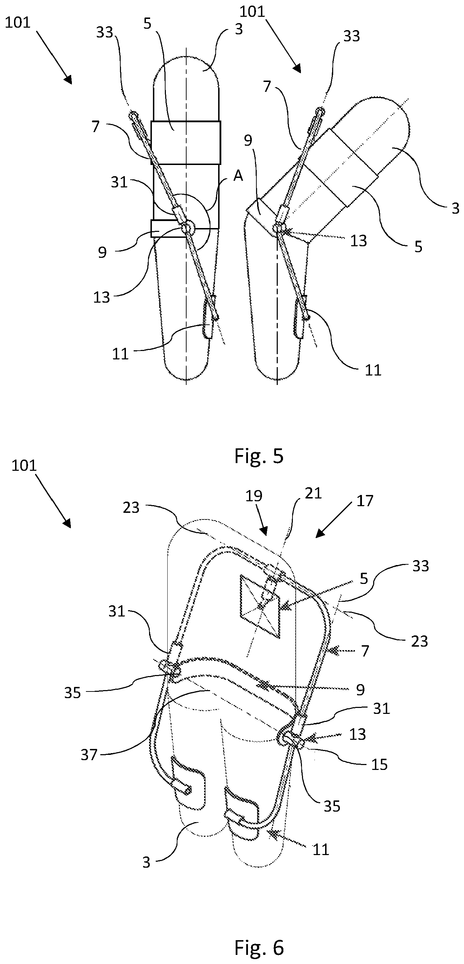

Fig. 5: a side view of human bodies wearing a wearable support structure according to the present disclosure are shown;
Fig. 6: an isometric view of a human body from Fig. 6 is shown;

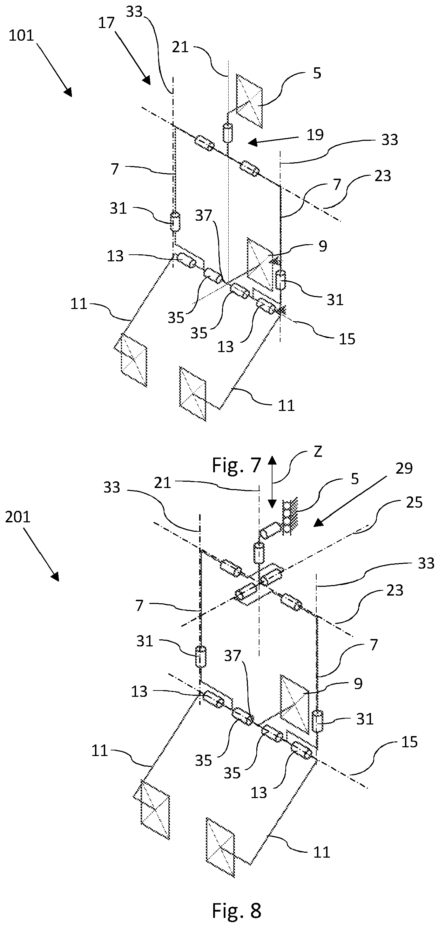

Fig. 7: a wearable support structure according to the present disclosure is shown;
Fig. 8: a further wearable support structure according to the present disclosure is shown;
Fig. 9: another wearable support structure according to the present disclosure is shown;

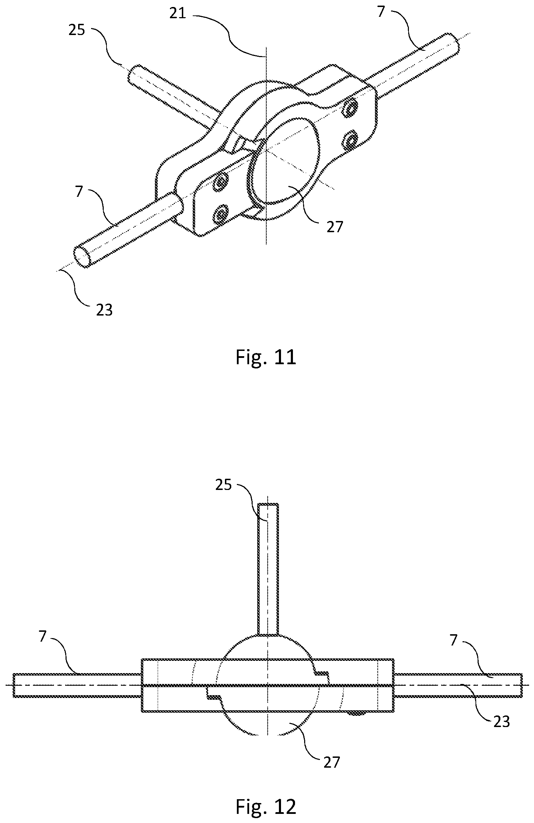

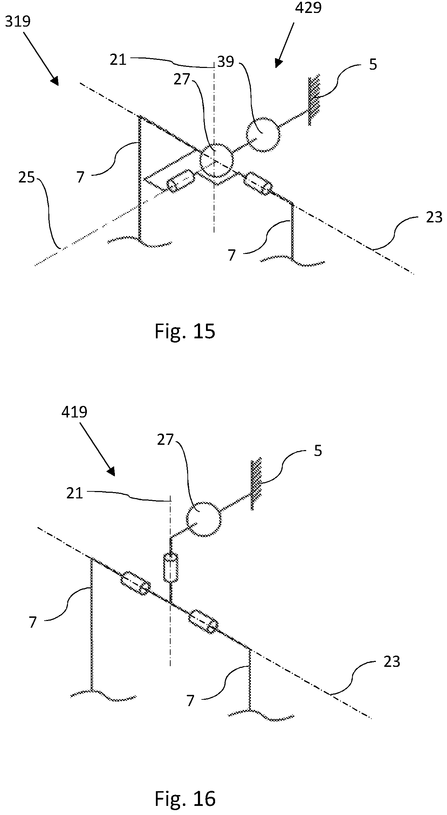

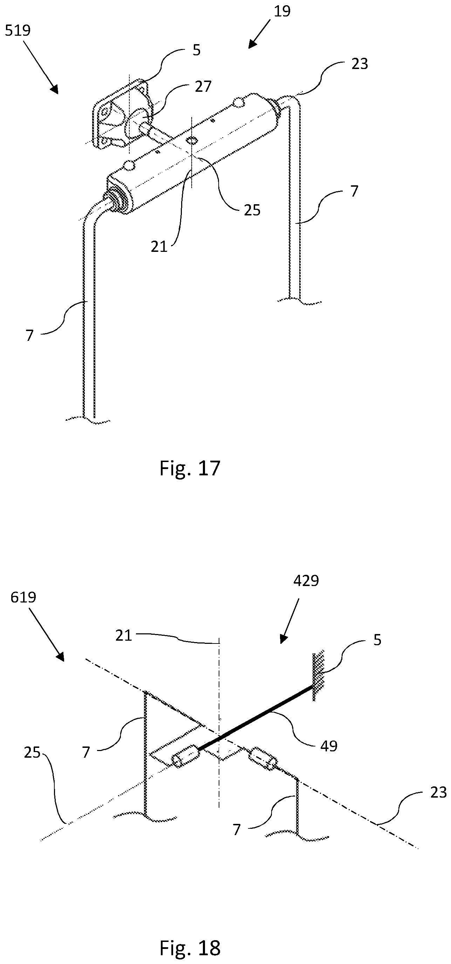

Fig. 10 - 18: elements of a wearable support structure according to the present disclosure are shown.

Wearable support structure 1 comprises a torso support 5 arranged for bearing upon a chest and a back of the human body 3. The torso support 5 comprises a belt that may be provided at the location of the chest around the torso of the human body 3. The torso support 5 attaches the wearable support structure 1 in a relative firm manner to the human body 3. Two elongated resilient stays 7 are provided and connected to said torso support 5. The two elongated resilient stays 7 extend substantially alongside the human body 3 and are arranged for being in a first state when being unloaded, and arranged to be biased back to said first state when being into a second state due to leaning or bending of the human body 3. The stays 7 may be substantially strip shaped, having a three-dimensional curved shape, the strip having for example a flat, curved, triangular or multi angular, round or oval cross section, which may be constant over the length of the stay or can vary along its length.

The wearable support structure 1 further comprises a lumbar support 9. The lumbar support 9 is arranged for bearing upon a lumbar area of said human body 3. At a lower end of the wearable support structure 1, the wearable support structure 1 is provided with two thigh supports 11. The thigh supports 11 are each arranged for bearing upon a front side of a respective thigh area of the human body 3. The thigh supports 11 may also be arranged for being in a first state when being unloaded, and arranged to be biased back to the first state when being into a second state due to leaning or bending of the human body 3. During leaning or bending the thigh supports 11 may press firmly against the thigh areas of the user.

A respective elongated resilient stay 7 of the two elongated resilient stays 7 and a respective thigh support 11 of the two thigh supports 11 are connected to a pivoting arrangement 13 of the wearable support structure 1. The pivoting arrangement 13 allows for pivoting the respective elongated resilient stay 7 and the respective thigh support 11 about a main axis 15. The main axes 15 of the pivoting arrangements 13 are concentric and preferably substantially parallel to a frontal axis of the human body 3 and substantially parallel to the axis over which the human body 3 bends forward. The main axis 15 may coincide with an axis through hip joints of the human body 3.

The lumbar support 9 is pivotally connected to the two pivoting arrangements 13 for pivoting, via a third pivot structure 35 of the wearable support structure 1, the lumbar support 9 about a fifth pivot axis 37. The fifth pivot axis 37 is concentric with the main axes 15 of the pivoting arrangements 13. The lumbar support 9 may be formed as a flexible belt that, at least during use of the wearable support structure 1, presses against the lumbar area of the user.

The wearable support structure 1 according to the present disclosure comprises a movement arrangement 17. The movement arrangement 17 is arranged for maintaining the two elongated resilient stays 7 and the two thigh supports 11 in their first states when the user of the wearable support structure 1 is walking. The movement arrangement 17 comprises a first pivot structure 19 and a second pivot structure 31.

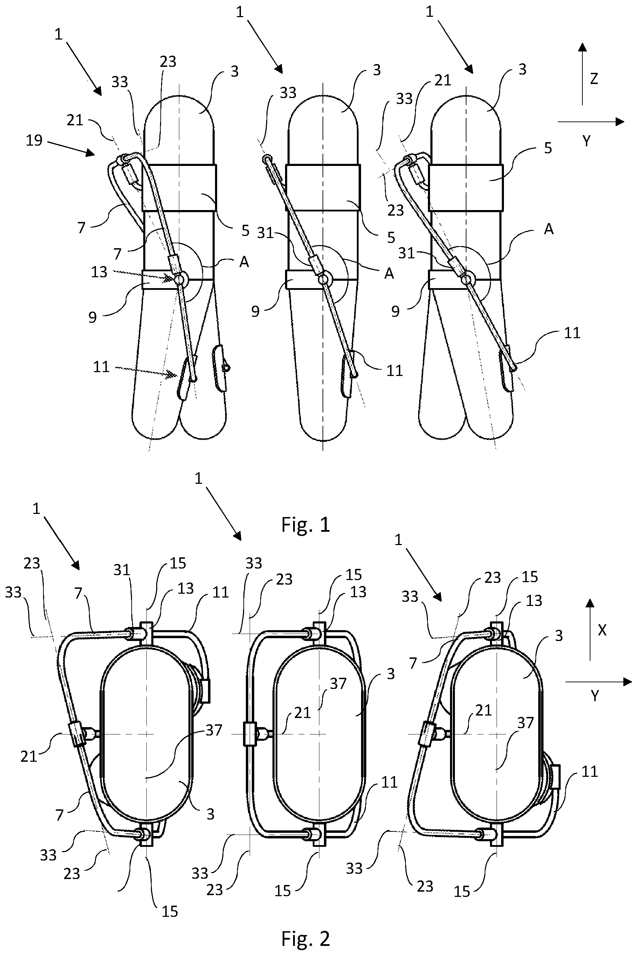

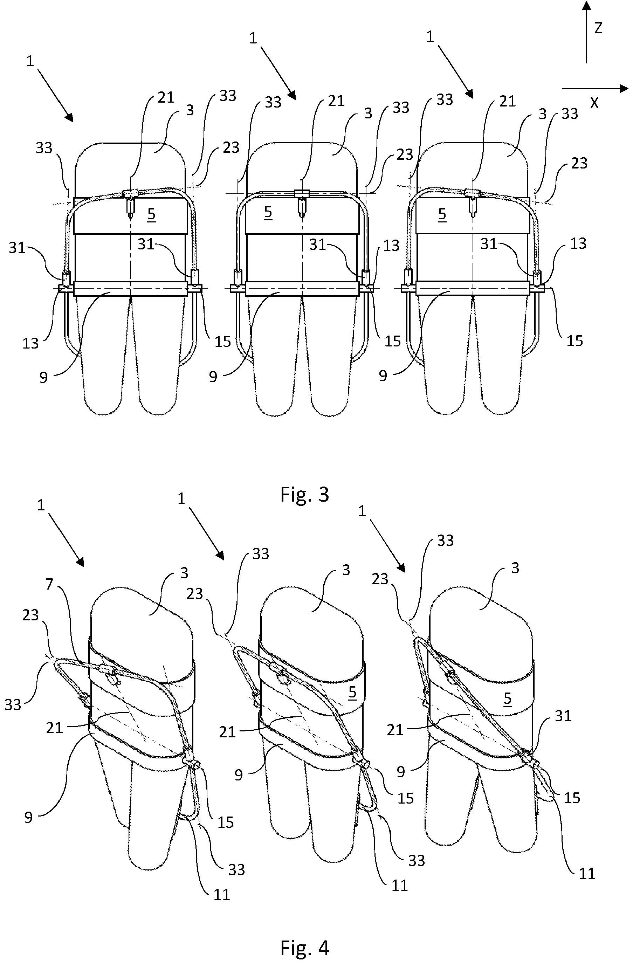

The first pivot structure 19 connects the two elongated resilient stays 7 to the torso support 5. The torso support 5 may pivot, via the first pivot structure 19, relative to the two elongated resilient stays 7 about a first pivot axis 21 and a second pivot axis 23. The first pivot axis 21 is at right angles to the second pivot axis 23. In addition, the first pivot axis 21 extends in a direction comprising a component in a vertical direction Z when the human body 3 is in the upright position shown in figures 1-4. The first pivot structure 19 is arranged for allowing the two elongated resilient stays 7 to pivot independent about the second pivot axis 23 from each other relative to the torso support 5.

The second pivot structure 31 connects the elongated resilient stays 7 to the pivoting arrangements 13 and allows each of the elongated resilient stays 7 to pivot about a fourth pivot axis 33 relative to the pivoting arrangement 13. The fourth pivot axis 33 extends along the elongated resilient stay 7 and is perpendicular to the main axis 15.

Wearable support structure 101 differs mainly from wearable support structure 1 in that the two pivoting arrangements 13 are arranged for allowing the respective elongated resilient stay 7, connected to a respective pivoting arrangement 13 of the two pivoting arrangements 13, to pivot about the main axis 15 relative to the respective thigh support 11, connected to the respective pivoting arrangement 13 of the two pivoting arrangements 13. The pivoting arrangements 13 of the wearable support structure 101 are each arranged to provide a biasing force biasing the respective elongated resilient stay 7 into a predetermined angle A relative to the respective thigh support 11. In addition, the movement arrangement 17 of wearable support structure 101 is arranged for maintaining the respective elongated resilient stay 7 into the predetermined angle A relative to the respective thigh support 11 and maintaining the two elongated resilient stays 7 in the first state when the human body 3 is in the upright position and moving such as walking. Elements of wearable support structure 101 similar to elements of wearable support structure 1 are indicated by the same reference numbers.

Wearable support structure 201 differs mainly from wearable support structure 101 in that the first pivot structure of wearable support structure 201 is further arranged for pivoting the torso support 5 relative to the two elongated resilient stays 7 about a third pivot axis 25. The third pivot axis 25 is at right angles to the first pivot axis 21 and the second pivot axis 23. In addition, the first pivot structure of wearable support structure 201 comprises a translation arrangement 29 arranged for allowing the torso support 5 to translate relative to the two elongated resilient stays 7 in a direction having a component in the vertical direction Z. Elements of wearable support structure 201 similar to elements of wearable support structure 1 and/or 101 are indicated by the same reference numbers.

Wearable support structure 301 differs mainly from wearable support structure 201 in that the second pivot structure 31 of the wearable support structure 301 is arranged for pivoting the elongated resilient stays 7 about a sixth pivot axis 41 relative to the pivoting arrangement 13. The sixth pivot axis 41 is at substantially right angles relative to the main axis 15 and the fourth axis 33. Elements of wearable support structure 301 similar to elements of wearable support structure 1, 101 and/or 201 are indicated by the same reference numbers.

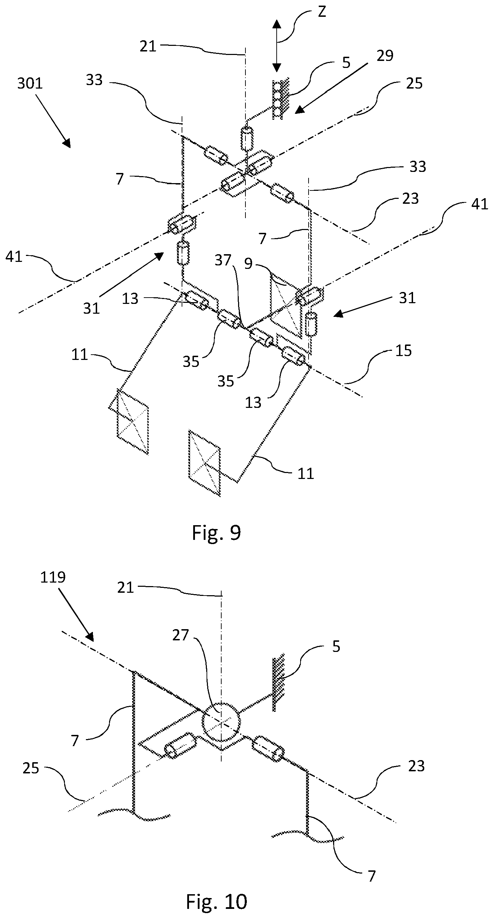

The first pivot structure 119 shown in Fig. 10 may be part of wearable support structures 1, 101, 201 or 301 and comprises a ball-joint coupling 27. The ball-joint coupling 27 allows for an omnidirectional pivoting of the torso support 5 relative to said two elongated resilient stays 7. The two elongated resilient stays 7 are mutually connected such that the two elongated resilient stays 7 may pivot relative to each other about the second pivot axis 23 and the third pivot axis 25. Elements of first pivot structure 119 that are similar to elements of first pivot structure 19 are indicated by the same reference numbers.

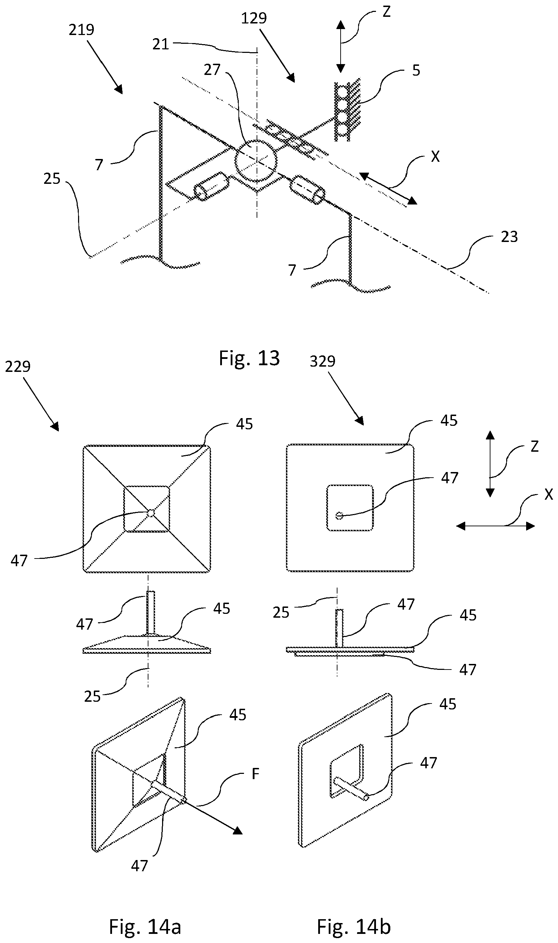

The first pivot structure 219 shown in Fig. 13 may be part of wearable support structures 1, 101, 201 or 301 and comprises a ball-joint coupling 27 and a translation arrangement 129. The translation arrangement 129 is arranged for allowing the torso support 5 to translate relative to the two elongated resilient stays 7 in directions X and Z. Elements of first pivot structure 219 that are similar to elements of first pivot structure 19 and/or 119 are indicated by the same reference numbers.

The translation arrangement 229 differs mainly from translation arrangement 129 in that translation arrangement 229 comprises a housing 45 and a translation part 47. The housing 45 is arranged for coupling the first pivot structure to the torso support 5. The translation part 47 may be displaced in the directions X and Z relative to the housing 45 for allowing the torso support 5 to translate relative to the two elongated resilient stays 7. The translation part 47 is received in a receiving space (not shown) of the housing 45. The receiving space and the translation part 47 are formed such that the translation part 47 is in a predetermine position when exerting a force F on the translation part 47 in direction Y, wherein direction Y is perpendicular to direction X and direction Z. Preferably, the predetermined position of the translation part 47 corresponds with a centre of the housing 45 in a virtual plane extending in the directions X and Z. Elements of translation arrangement 229 that are similar to elements of translation arrangement 29 and/or 129 are indicated by the same reference numbers.

The translation arrangement 329 differs mainly from translation arrangement 229 in that the translation arrangement 329 is arranged for translating the torso support 5 in two perpendicular directions X and Z in a flat virtual plane relative to the two elongated resilient stays 7. Elements of translation arrangement 329 that are similar to elements of translation arrangement 29, 129 and/or 229 are indicated by the same reference numbers.

The first pivot structure 319 may be part of wearable support structures 1, 101, 201 or 301. The first pivot structure 319 differs mainly from first pivot structure 119 in that the first pivot structure 319 comprises a further ball-joint coupling 39. The ball-joint coupling 27 is connected to the torso support 5 via the further ball-joint coupling 39. The translation arrangement 429 of first pivot structure 319 comprises the ball-joint coupling 27 and the further ball-joint coupling 39. Elements of first pivot structure 319 that are similar to elements of first pivot structure 19, 119 and/or 219 are indicated by the same reference numbers. The first pivot structure 419 may be part of wearable support structures 1, 101, 201 or 301. The first pivot structure 419 differs mainly from first pivot structure 19 in that the first pivot structure 419 comprises a ball-joint coupling 27. Elements of first pivot structure 419 that are similar to elements of first pivot structure 19, 119, 219 and/or 319 are indicated by the same reference numbers.

The first pivot structure 519 may be part of wearable support structures 1, 101, 201 or 301. The first pivot structure 519 differs mainly from first pivot structure 19 in that the first pivot structure 519 comprises a ball-joint coupling 27. Elements of first pivot structure 519 that are similar to elements of first pivot structure 19, 119, 219, 319 and/or 419 are indicated by the same reference numbers.

The first pivot structure 619 may be part of wearable support structures 1, 101, 201 or 301. The first pivot structure 619 differs mainly from first pivot structure 319 in that the first pivot structure 619 comprises a flexible element 49, such as a cable or a wire, wherein the omnidirectional pivot coupling and the further omnidirectional pivot coupling are formed by the flexible element 49. Elements of first pivot structure 619 that are similar to elements of first pivot structure 19, 119, 219, 319, 419 and/or 519 are indicated by the same reference numbers.

## Claims

1. Wearable support structure (1, 101, 201, 301) for at least partly relieving a human body (3) during leaning or bending over, said wearable support structure (1, 101, 201, 301) comprising:
- a torso support (5) arranged for bearing upon a chest and/or a back of said human body (3);
- two elongated resilient stays (7), connected to said torso support (5), arranged to extend substantially alongside said human body (3) and arranged for being in a first state when being unloaded, and arranged to be biased back to said first state when being into a second state corresponding with a leaning or bending position of said human body (3);
- a lumbar support (9) arranged for bearing upon a lumbar area of said human body (3);
- two thigh supports (11), each arranged for bearing upon a respective thigh area of said human body (3), arranged for being in a first state when being unloaded, and arranged to be biased back to said first state when being into a second state corresponding with a leaning or bending position of said human body (3);
- two pivoting arrangements (13) allowing said respective elongated resilient stay (7) to pivot about a main axis (15),
wherein said wearable support structure (1, 101, 201, 301) comprises a movement arrangement (17) arranged for maintaining said two elongated resilient stays (7) and said two thigh supports (11) in said first states when said human body (3) is in an upright position,
**characterized in that** said two pivoting arrangements (13) tolt each connect a respective elongated resilient stay (7) of said two elongated resilient stays (7) to a respective thigh support (11) of said two thigh supports (11) and allow said respective thigh support (11) to pivot about the main axis (15) and **in that** the movement arrangement (17) is arranged for maintaining a pivot angle about said main axis (15) of said respective elongated resilient stay (7) of said two elongated resilient stays (7) substantially equal to a pivot angle about said main axis (15) of said respective thigh support (11) of said two thigh supports (11) when said human body (3) is in said upright position.

2. Wearable support structure (1, 101, 201, 301) according to claim 1, wherein said movement arrangement (17) comprises a first pivot structure (19, 119, 219, 319, 419, 519), wherein said torso support (5) is pivotally connected, via said first pivot structure (19, 119, 219, 319, 419, 519) to said two elongated resilient stays (7).

3. Wearable support structure (1, 101, 201, 301) according to claim 2, wherein said first pivot structure (19, 119, 219, 319, 419, 519) is arranged for pivoting said torso support (5) relative to said two elongated resilient stays (7) about a first pivot axis (21) and a second pivot axis (23), wherein said first pivot axis (21) is at right angles to said second pivot axis (23) and wherein said first pivot axis (21), when said torso support (5) is bearing upon said chest and/or said back of said human body (3), extends in a direction comprising a component in a vertical direction when said human body (3) is in said upright position.

4. Wearable support structure (201, 301) according to claim 3, wherein said first pivot structure (19, 119, 219, 319, 419, 519) is further arranged for pivoting said torso support (5) relative to said two elongated resilient stays (7) about a third pivot axis (25), wherein said third pivot axis (25) is at right angles to said first pivot axis (21) and said second pivot axis (23).

5. Wearable support structure according to claim 2, 3 or 4, wherein said first pivot structure (119, 219, 319, 419, 519) comprises an omnidirectional pivot coupling, preferably a ball-joint coupling (27), for allowing omnidirectional pivoting of said torso support (5) relative to said two elongated resilient stays (7).

6. Wearable support structure (201, 301) according to any one of the claims 2 to 5, wherein said first pivot structure (219, 319) comprises a translation arrangement (29, 129, 229, 329, 429) arranged for allowing said torso support (5) to translate relative to said two elongated resilient stays (7).

7. Wearable support structure according to claims 5 and 6, wherein said translation arrangement (429) comprises a further omnidirectional pivot coupling, preferably a further ball-joint (39), for allowing said torso support (5) to translate relative to said two elongated resilient stays (7), preferably for allowing said torso support (5) to translate over a predetermined distance relative to said two elongated resilient stays (7).

8. Wearable support structure according to claim 6 or 7, wherein said translation arrangement (329) is arranged for translating said torso support (5) in two perpendicular directions (X, Z) in a flat virtual plane relative to said two elongated resilient stays (7).

9. Wearable support structure (1, 101, 201, 301) according to any one of the claims 2 to 8, wherein said movement arrangement (17) is arranged for allowing said two elongated resilient stays (7) to pivot independent from each other relative to said torso support (5).

10. Wearable support structure (1, 101, 201, 301) according to any one of the claims 2 to 9, wherein said movement arrangement (17) further comprises a second pivot structure (31) for allowing each of said elongated resilient stays (7) to pivot about a fourth pivot axis (33) relative to said pivoting arrangement (13) connecting said elongated resilient stay (7), via said pivoting arrangement (13), to said thigh support (11), wherein said fourth pivot axis (33) extends along said elongated resilient stay (7).

11. Wearable support structure (1, 101, 201, 301) according to claim 10, wherein said fourth pivot axis (33) is perpendicular to said main axis (15).

12. Wearable support structure (1, 101, 201, 301) according to any one of the preceding claims, wherein said main axes (15) of said pivoting arrangements (13) are concentric.

13. Wearable support structure (1, 101, 201, 301) according to any one of the preceding claims, wherein said wearable support structure (1, 101, 201, 301) comprises a third pivot structure (35), wherein said lumbar support (9) is pivotally connected, via said third pivot structure (35), to said two pivoting arrangements (13).

14. Wearable support structure (1, 101, 201, 301) according to claim 13, wherein said third pivot structure (35) is arranged for pivoting said lumbar support (9) relative to said two pivoting arrangements (13) about a fifth pivot axis (37), preferably wherein said fifth pivot axis (37) is concentric with said main axes (15).

15. Wearable support structure (101, 201, 301) according to any one of the preceding claims, wherein said two pivoting arrangements (13) are arranged for allowing said respective elongated resilient stay (7), connected to a respective pivoting arrangement (13) of said two pivoting arrangements (13), to pivot about said main axis (15) relative to said respective thigh support (11), connected to said respective pivoting arrangement (13) of said two pivoting arrangements (13), wherein each of said two pivoting arrangements (13) is arranged to provide a biasing force biasing said respective elongated resilient stay (7) into a predetermined angle (A) relative to said respective thigh support (11), wherein said movement arrangement (17) is arranged for maintaining said respective elongated resilient stay (7) into said predetermined angle (A) relative to said respective thigh support (11) and maintaining said two elongated resilient stays (7) in said first state when said human body (3) is in said upright position.

## Patentansprüche

1. Tragbare Stützstruktur (1, 101, 201, 301) zur zumindest teilweisen Entlastung eines menschlichen Körpers (3) beim Vorbeugen oder Bücken, wobei die tragbare Stützstruktur (1, 101, 201, 301) umfasst:
- eine Rumpfstütze (5), die zum Aufliegen auf der Brust und/oder dem Rücken des menschlichen Körpers (3) angeordnet ist;
- zwei langgestreckte elastische Streben (7), die mit der Rumpfstütze (5) verbunden und so angeordnet sind, dass sie sich im Wesentlichen entlang des menschlichen Körpers (3) erstrecken und so angeordnet sind, dass sie sich in einem ersten Zustand befinden, wenn sie unbelastet sind, und dazu angeordnet sind, in den ersten Zustand zurückzufedern, wenn sie sich in einem zweiten Zustand befinden, der einer Vorbeuge- oder Rückstellung des menschlichen Körpers (3) entspricht;
- eine Lendenstütze (9), die zum Aufliegen auf dem Lendenwirbelbereich des menschlichen Körpers (3) angeordnet ist;
- zwei Oberschenkelstützen (11), jeweils zum Aufliegen auf einem entsprechenden Oberschenkelbereich des menschlichen Körpers (3) angeordnet, die dazu angeordnet sind, sich in einem ersten Zustand zu befinden, wenn sie nicht belastet werden, und die dazu angeordnet sind, in den ersten Zustand zurückzufedern, wenn sie sich in einem zweiten Zustand befinden, der einer Vorbeuge- oder Rückstellung des menschlichen Körpers (3) entspricht.;
- zwei Schwenkanordnungen (13), die es den jeweiligen langgestreckten, elastischen Streben (7) ermöglichen, um eine Hauptachse (15) zu schwenken,
wobei die tragbare Stützstruktur (1, 101, 201, 301) eine Bewegungsanordnung (17) aufweist, die dazu angeordnet ist, die zwei langgestreckten, elastischen Streben (7) und die zwei Oberschenkelstützen (11) in den ersten Zuständen zu halten, wenn sich der menschliche Körper (3) in einer aufrechten Stellung befindet,
**gekennzeichnet dadurch, dass** die zwei Schwenkanordnungen (13) jeweils eine jeweilige langgestreckte, elastische Strebe (7) der zwei langgestreckten, elastischen Streben (7) mit einer jeweiligen Oberschenkelstütze (11) der zwei Oberschenkelstützen (11) verbinden und es der jeweiligen Oberschenkelstütze (11) ermöglichen, um die Hauptachse (15) zu schwenken, und dadurch, dass die Bewegungsanordnung (17) dazu angeordnet ist, einen Schwenkwinkel um die Hauptachse (15) der jeweiligen langgestreckten, elastischen Strebe (7) der zwei langgestreckten, elastischen Streben (7) im Wesentlichen gleich einem Schwenkwinkel um die Hauptachse (15) der jeweiligen Oberschenkelstütze (11) der zwei Oberschenkelstützen (11) zu halten, wenn sich der menschliche Körper (3) in der aufrechten Stellung befindet.

2. Tragbare Stützstruktur (1, 101, 201, 301) nach Anspruch 1, wobei die Bewegungsanordnung (17) eine erste Schwenkstruktur (19, 119, 219, 319, 419, 519) umfasst, wobei die Rumpfstütze (5) über die erste Schwenkstruktur (19, 119, 219, 319, 419, 519) schwenkbar mit den zwei langgestreckten, elastischen Streben (7) verbunden ist.

3. Tragbare Stützstruktur (1, 101, 201, 301) nach Anspruch 2, wobei die erste Schwenkstruktur (19, 119, 219, 319, 419, 519) dazu angeordnet ist, die Rumpfstütze (5) relativ zu den zwei langgestreckten, elastischen Streben (7) um eine erste Schwenkachse (21) und eine zweite Schwenkachse (23) zu schwenken, wobei die erste Schwenkachse (21) rechtwinklig zur zweiten Schwenkachse (23) steht und wobei die erste Schwenkachse (21), wenn die Rumpfstütze (5) auf der Brust und/oder dem Rücken des menschlichen Körpers (3) aufliegt, sich in eine Richtung erstreckt, die eine Komponente in vertikaler Richtung aufweist, wenn sich der menschliche Körper (3) in der aufrechten Stellung befindet.

4. Tragbare Stützstruktur (201, 301) nach Anspruch 3, wobei die erste Schwenkstruktur (19, 119, 219, 319, 419, 519) ferner dazu angeordnet ist, die Rumpfstütze (5) relativ zu den zwei langgestreckten, elastischen Streben (7) um eine dritte Schwenkachse (25) zu schwenken, wobei die dritte Schwenkachse (25) rechtwinklig zur ersten Schwenkachse (21) und zur zweiten Schwenkachse (23) steht.

5. Tragbare Stützstruktur nach Anspruch 2, 3 oder 4, wobei die erste Schwenkstruktur (119, 219, 319, 419, 519) eine omnidirektionale Drehkupplung, vorzugsweise eine Kugelgelenkkupplung (27), umfasst, um ein omnidirektionales Schwenken der Rumpfstütze (5) relativ zu den zwei langgestreckten, elastischen Streben (7) zu ermöglichen.

6. Tragbare Stützstruktur (201, 301) nach einem der Ansprüche 2 bis 5, wobei die erste Schwenkstruktur (219, 319) eine Verschiebeanordnung (29, 129, 229, 329, 429) umfasst, die dazu angeordnet ist, dass die Rumpfstütze (5) sich relativ zu den zwei langgestreckten, elastischen Streben (7) verschieben kann.

7. Tragbare Stützstruktur nach den Ansprüchen 5 und 6, wobei die Verschiebeanordnung (429) eine weitere omnidirektionale Drehkupplung, vorzugsweise ein weiteres Kugelgelenk (39), umfasst, um der Rumpfstütze (5) ein Verschieben relativ zu den zwei langgestreckten, elastischen Streben (7) zu ermöglichen, vorzugsweise um der Rumpfstütze (5) ein Verschieben über eine vorbestimmte Entfernung relativ zu den zwei langgestreckten, elastischen Streben (7) zu ermöglichen.

8. Tragbare Stützstruktur nach Anspruch 6 oder 7, wobei die Verschiebeanordnung (329) dazu angeordnet ist, die Rumpfstütze (5) in zwei rechtwinkligen Richtungen (X, Z) in einer flachen virtuellen Ebene relativ zu den zwei langgestreckten, elastischen Streben (7) zu verschieben.

9. Tragbare Stützstruktur (1, 101, 201, 301) nach einem der Ansprüche 2 bis 8, wobei die Bewegungsanordnung (17) dazu angeordnet ist, dass die zwei langgestreckten, elastischen Streben (7) unabhängig voneinander relativ zur Rumpfstütze (5) schwenkbar sind.

10. Tragbare Stützstruktur (1, 101, 201, 301) nach einem der Ansprüche 2 bis 9, wobei die Bewegungsanordnung (17) ferner eine zweite Schwenkstruktur (31) umfasst, um jeder der langgestreckten, elastischen Streben (7) zu ermöglichen, um eine vierte Schwenkachse (33) relativ zur der Schwenkanordnung (13) zu schwenken, die die langgestreckte, elastische Strebe (7) über die Schwenkanordnung (13) mit der Oberschenkelstütze (11) verbindet, wobei sich die vierte Schwenkachse (33) entlang der langgestreckten, elastischen Strebe (7) erstreckt.

11. Tragbare Stützstruktur (1, 101, 201, 301) nach Anspruch 10, wobei die vierte Schwenkachse (33) rechtwinklig zur Hauptachse (15) steht.

12. Tragbare Stützstruktur (1, 101, 201, 301) nach einem der vorhergehenden Ansprüche, wobei die Hauptachsen (15) der Schwenkanordnungen (13) konzentrisch sind.

13. Tragbare Stützstruktur (1, 101, 201, 301) nach einem der vorhergehenden Ansprüche, wobei die tragbare Stützstruktur (1, 101, 201, 301) eine dritte Schwenkstruktur (35) aufweist, wobei die Lendenstütze (9) über die dritte Schwenkstruktur (35) schwenkbar mit den zwei Schwenkanordnungen (13) verbunden ist.

14. Tragbare Stützstruktur (1, 101, 201, 301) nach Anspruch 13, wobei die dritte Schwenkstruktur (35) dazu angeordnet ist, die Lendenstütze (9) relativ zu den zwei Schwenkanordnungen (13) um eine fünfte Schwenkachse (37) zu schwenken, vorzugsweise wobei die fünfte Schwenkachse (37) mit den Hauptachsen (15) konzentrisch ist.

15. Tragbare Stützstruktur (101, 201, 301) nach einem der vorhergehenden Ansprüche, wobei die zwei Schwenkanordnungen (13) dazu angeordnet sind, dass die jeweilige langgestreckte, elastische Strebe (7), die mit einer jeweiligen Schwenkanordnung (13) der zwei Schwenkanordnungen (13) verbunden ist, um die Hauptachse (15) relativ zu der jeweiligen Oberschenkelstütze (11) schwenken kann, die mit der jeweiligen Schwenkanordnung (13) der zwei Schwenkanordnungen (13) verbunden ist, wobei jede der zwei Schwenkanordnungen (13) dazu angeordnet ist, eine Vorspannkraft bereitzustellen, die die jeweilige langgestreckte, elastische Strebe (7) in einen vorbestimmten Winkel (A) relativ zu der jeweiligen Oberschenkelstütze (11) vorspannt, wobei die Bewegungsanordnung (17) dazu angeordnet ist, die jeweilige langgestreckte, elastische Strebe (7) in dem vorbestimmten Winkel (A) relativ zu der jeweiligen Oberschenkelstütze (11) zu halten und die zwei langgestreckten, elastischen Streben (7) im ersten Zustand zu halten, wenn sich der menschliche Körper (3) in der aufrechten Stellung befindet.

## Revendications

1. Structure portable de support (1, 101, 201, 301) pour soulager au moins partiellement un corps humain (3) penché ou fléchi, ladite Structure portable de support (1, 101, 201, 301) comprenant :
- un support de torse (5) agencé pour venir en appui sur la poitrine et/ou le dos dudit corps humain (3) ;
- deux renforts élastiques allongés (7), reliés audit support de torse (5), agencés pour s'étendre sensiblement le long dudit corps humain (3) et agencés pour être dans un premier état lorsqu'ils ne sont pas chargés, et agencés pour être ramenés audit premier état lorsqu'ils sont dans un second état correspondant à une position penchée ou fléchie dudit corps humain (3) ;
- un support lombaire (9) agencé pour venir en appui sur une zone lombaire dudit corps humain (3) ;
- deux supports de cuisse (11), chacun agencé pour venir en appui sur une zone de cuisse respective dudit corps humain (3), agencés pour être dans un premier état lorsqu'ils ne sont pas chargés, et agencés pour être ramenés audit premier état lorsqu'ils sont dans un second état correspondant à une position penchée ou fléchie dudit corps humain (3) ;
- deux agencements de pivotement (13) permettant audit renfort élastique allongé respectif (7) de pivoter autour d'un axe principal (15),
dans laquelle ladite structure portable de support (1, 101, 201, 301) comprend un agencement de déplacement (17) agencé pour maintenir lesdits deux renforts élastiques allongés (7) et lesdits deux supports de cuisse (11) dans lesdits premiers états lorsque ledit corps humain (3) est dans une position verticale,
**caractérisée en ce que** lesdits deux agencements de pivotement (13) relient chacun un renfort élastique allongé respectif (7) desdits deux renforts élastiques allongés (7) à un support de cuisse respectif (11) desdits deux supports de cuisse (11) et permettent audit support de cuisse respectif (11) de pivoter autour de l'axe principal (15) et **en ce que** l'agencement de déplacement (17) est agencé pour maintenir un angle de pivotement autour dudit axe principal (15) dudit renfort élastique allongé respectif (7) desdits deux renforts élastiques allongés (7) sensiblement égal à un angle de pivotement autour dudit axe principal (15) dudit support de cuisse respectif (11) desdits deux supports de cuisse (11) lorsque ledit corps humain (3) est dans ladite position verticale.

2. Structure portable de support (1, 101, 201, 301) selon la revendication 1, dans laquelle ledit agencement de déplacement (17) comprend une première structure pivotante (19, 119, 219, 319, 419, 519), dans laquelle ledit support de torse (5) est relié en pivotement, via ladite première structure pivotante (19, 119, 219, 319, 419, 519), auxdits deux renforts élastiques allongés (7).

3. Structure portable de support (1, 101, 201, 301) selon la revendication 2, dans laquelle ladite première structure pivotante (19, 119, 219, 319, 419, 519) est agencée pour faire pivoter ledit support de torse (5) par rapport auxdits deux renforts élastiques allongés (7) autour d'un premier axe de pivotement (21) et d'un deuxième axe de pivotement (23), dans laquelle ledit premier axe de pivotement (21) est perpendiculaire audit deuxième axe de pivotement (23) et dans laquelle ledit premier axe de pivotement (21), lorsque ledit support de torse (5) prend appui sur ladite poitrine et/ou ledit dos dudit corps humain (3), s'étend dans une direction comprenant une composante dans une direction verticale lorsque ledit corps humain (3) est dans ladite position verticale.

4. Structure portable de support (201, 301) selon la revendication 3, dans laquelle ladite première structure pivotante (19, 119, 219, 319, 419, 519) est en outre agencée pour faire pivoter ledit support de torse (5) par rapport auxdits deux renforts élastiques allongés (7) autour d'un troisième axe de pivotement (25), dans laquelle ledit troisième axe de pivotement (25) est perpendiculaire audit premier axe de pivotement (21) et audit deuxième axe de pivotement (23).

5. Structure portable de support selon l'une quelconque des revendications 2, 3 ou 4, dans laquelle ladite première structure pivotante (119, 219, 319, 419, 519) comprend un couplage pivotant omnidirectionnel, de préférence un couplage à rotule (27), pour permettre un pivotement omnidirectionnel dudit support de torse (5) par rapport auxdits deux renforts élastiques allongés (7).

6. Structure portable de support (201, 301) selon l'une quelconque des revendications 2 à 5, dans laquelle ladite première structure pivotante (219, 319) comprend un agencement de translation (29, 129, 229, 329, 429) agencé pour permettre audit support de torse (5) d'effectuer une translation par rapport auxdits deux renforts élastiques allongés (7).

7. Structure portable de support selon les revendications 5 et 6, dans laquelle ledit agencement de translation (429) comprend un couplage pivotant omnidirectionnel supplémentaire, de préférence une rotule supplémentaire (39), pour permettre audit support de torse (5) d'effectuer une translation par rapport auxdits deux renforts élastiques allongés (7), de préférence pour permettre audit support de torse (5) d'effectuer une translation sur une distance prédéterminée par rapport auxdits deux renforts élastiques allongés (7).

8. Structure portable de support selon la revendication 6 ou 7, dans laquelle ledit agencement de translation (329) est agencé pour entraîner en translation ledit support de torse (5) dans deux directions perpendiculaires (X, Z) dans un plan virtuel plat par rapport auxdits deux renforts élastiques allongés (7).

9. Structure portable de support (1, 101, 201, 301) selon l'une quelconque des revendications 2 à 8, dans laquelle ledit agencement de déplacement (17) est agencé pour permettre auxdits deux renforts élastiques allongés (7) de pivoter indépendamment l'un de l'autre par rapport audit support de torse (5).

10. Structure portable de support (1, 101, 201, 301) selon l'une quelconque des revendications 2 à 9, dans laquelle ledit agencement de déplacement (17) comprend en outre une deuxième structure pivotante (31) pour permettre à chacun desdits renforts élastiques allongés (7) de pivoter autour d'un quatrième axe de pivotement (33) par rapport audit agencement de pivotement (13) reliant ledit renfort élastique allongé (7), via ledit agencement de pivotement (13), audit support de cuisse (11), dans laquelle ledit quatrième axe de pivotement (33) s'étend le long dudit renfort élastique allongé (7).

11. Structure portable de support (1, 101, 201, 301) selon la revendication 10, dans laquelle ledit quatrième axe de pivotement (33) est perpendiculaire audit axe principal (15).

12. Structure portable de support (1, 101, 201, 301) selon l'une quelconque des revendications précédentes, dans laquelle lesdits axes principaux (15) desdits agencements de pivotement (13) sont concentriques.

13. Structure portable de support (1, 101, 201, 301) selon l'une quelconque des revendications précédentes, dans laquelle ladite structure portable de support (1, 101, 201, 301) comprend une troisième structure pivotante (35), dans laquelle ledit support lombaire (9) est relié en pivotement, via ladite troisième structure pivotante (35), auxdits deux agencements de pivotement (13).

14. Structure portable de support (1, 101, 201, 301) selon la revendication 13, dans laquelle ladite troisième structure pivotante (35) est agencée pour faire pivoter ledit support lombaire (9) par rapport auxdits deux agencements de pivotement (13) autour d'un cinquième axe de pivotement (37), de préférence dans laquelle ledit cinquième axe de pivotement (37) est concentrique auxdits axes principaux (15).

15. Structure portable de support (101, 201, 301) selon l'une quelconque des revendications précédentes, dans laquelle lesdits deux agencements de pivotement (13) sont agencés pour permettre audit renfort élastique allongé respectif (7), relié à un agencement de pivotement respectif (13) desdits deux agencements de pivotement (13), de pivoter autour dudit axe principal (15) par rapport audit support de cuisse respectif (11), relié audit agencement de pivotement respectif (13) desdits deux agencements de pivotement (13), dans laquelle chacun desdits deux agencements de pivotement (13) est agencé pour fournir une force de sollicitation sollicitant ledit renfort élastique allongé respectif (7) selon un angle prédéterminé (A) par rapport audit support de cuisse respectif (11), dans laquelle ledit agencement de déplacement (17) est agencé pour maintenir ledit renfort élastique allongé respectif (7) selon ledit angle prédéterminé (A) par rapport audit support de cuisse respectif (11) et maintenir lesdits deux renforts élastiques allongés (7) dans ledit premier état lorsque ledit corps humain (3) est dans ladite position verticale.
